# FASCICULE DE BREVET EUROPEEN

(11) **EP 2 877 094 B1**
(45) Date de publication et mention de la délivrance du brevet: **08.09.2021**
(21) Numéro de dépôt: 13756588.3
(22) Date de dépôt: 23.07.2013
(51) Int. Cl.: A61B 6/10, A61B 6/00, A61B 50/13

(54) **PARAVENT EN MATÉRIAU RADIOPROTECTEUR POUR LA PROTECTION D'UN OPÉRATEUR À L'ENCONTRE DES RAYONNEMENTS IONISANTS**
SCHIRM AUS EINEM STRAHLENSCHUTZMATERIAL ZUM SCHUTZ EINES BEDIENERS VOR IONISIERENDER STRAHLUNG
SCREEN MADE OF RADIATION SHIELDING MATERIAL FOR PROTECTING AN OPERATOR FROM IONISING RADIATION

(30) Priorité: 24.07.2012 FR 1257176
(43) Date de publication de la demande: 03.06.2015
(73) Titulaire: Lemer Pax, 44240 La Chapelle-sur-Erdre (FR)
(72) Inventeur: LEMER, Pierre-Marie, 44000 Nantes (FR)
(74) Mandataire: Jacobacci Coralis Harle
(86) Numéro de dépôt international: PCT/FR2013/051778
(87) Numéro de publication internationale: WO 2014/016513

(56) Documents cités:
- WO-A2-2009/156660
- FR-A1- 2 915 868
- US-A1- 2008 217 564
- US-A1- 2012 049 093
- None

## Description

La présente invention concerne les paravents radioprotecteurs, et plus particulièrement les paravents qui sont utilisés en milieu médical ou autre, pour protéger un opérateur à l'encontre des émissions de rayonnements ionisants, par exemple les rayons X. Elle concerne également un équipement en forme de housse stérile pour l'habillage par recouvrement de tels paravents, en vue de leur utilisation dans une ambiance protégée stérile.

Dans le cadre de certains examens ou interventions, on soumet les patients à des rayonnements ionisants, type rayons X notamment, utilisés dans un but de contrôle, de diagnostic ou de traitement.

C'est le cas en particulier pour des interventions du genre cathétérisme, pose de pacemakers, examens vasculaires, neurologiques ou urologiques, CRM (Cardiac Rhythm Management), CRT (Cardiac Resynchronization Therapy)...

Il est alors important de protéger correctement l'opérateur (technicien, médecin, chirurgien ou autre) contre ces rayonnements, sous peine de l'exposer à des doses importantes, cumulées dans le temps, susceptibles d'engendrer des pathologies diverses (nécroses des membres supérieurs, tumeurs cérébrales etc.).

Il existe des structures de protection consistant en des vêtements du genre blouses, chasubles ou tabliers en matériau radioprotecteur, mais qui ne couvrent pas toujours la totalité du corps et dont le poids important nuit au confort de l'opérateur, limite ses capacités de mouvements et entraîne une fatigue rapide.

Il existe encore des écrans ou paravents constitués de panneaux ou d'assemblages de panneaux en matériau radioprotecteur approprié, posés directement sur le sol ou par l'intermédiaire d'une embase support, par exemple un châssis roulant.

Par exemple, le document US 2012/0049093 décrit une structure de paravent dont une partie verticale est destinée à venir se positionner sous un plateau support adapté pour la réception d'un patient, et dont une autre partie verticale, portée par la première, est destinée à venir se positionner par-dessus ledit plateau support et le patient.

Cette seconde partie est montée mobile verticalement par rapport à la première, en particulier pour faciliter le positionnement du paravent au plus près du corps du patient allongé sur le plateau.

Mais une telle structure n'est aucunement adaptée pour permettre à un opérateur situé du côté protégé du paravent, d'accéder, par ses bras et ses mains, de l'autre côté de ce paravent, par exemple pour intervenir sur une partie du corps du patient exposée aux rayonnements.

D'autres écrans ou paravents radioprotecteurs connus, par exemple tels que décrits dans les documents FR-2 915 868, WO-2009/156660, US2008/0217564, ou encore US-3 308 297, consistent en des structures bien adaptées pour protéger un opérateur intervenant sur un patient exposé aux rayonnements, cela par l'intermédiaire d'orifice(s) ou d'ouverture(s) pour le passage de ses bras et de ses mains.

Mais les structures actuelles de ce genre ne permettent pas toujours à l'opérateur de travailler dans des conditions optimales.

En particulier, certaines interventions comportent des phases ou étapes intermédiaires au cours desquelles l'opérateur ne nécessite pas de radioprotection, mais a besoin d'une grande liberté de mouvement, par exemple pour la pose de dispositifs cardiaques implantables, pacemakers, défibrillateurs etc.

La présence du paravent est alors relativement gênante. L'opérateur doit le contourner ou alors le paravent doit être écarté temporairement, ce qui n'est pas pratique à mettre en œuvre et ce qui pose des problèmes de place, d'organisation, voire même de sécurité au sein de la salle d'intervention.

Le but de la présente invention est de proposer une nouvelle structure de paravent radioprotecteur au travers de laquelle un intervenant peut passer ses mains, et éventuellement ses bras, pour intervenir sur un patient, tout en ayant le reste de son corps efficacement protégé contre les rayonnements, cette structure de paravent étant, apte à être conformée très facilement selon une configuration de radioprotection optimale, mais aussi selon une configuration dans laquelle l'opérateur dispose d'une liberté de mouvement plus importante, en particulier pour ce qui concerne ses membres supérieurs, au regard de l'accès au patient ou au proche environnement de ce dernier.

La présente invention est définie par le paravent en matériau radioprotecteur selon la revendication 1.

Selon une caractéristique, ledit passage pour les bras de l'opérateur est ménagé au niveau de la zone de juxtaposition entre lesdites parties inférieure et supérieure, lorsque ces dernières sont en position juxtaposée active.

Dans ce cas, ledit passage pour les bras de l'opérateur est avantageusement ménagé au niveau de la bordure inférieure de la paroi frontale de la partie supérieure, et il consiste en un rideau souple en forme d'une pluralité de bandes ou lanières verticales juxtaposées, réalisées en matériau radioprotecteur, et dont l'extrémité supérieure est fixée sur un panneau supérieur réalisé en matériau radioprotecteur.

La partie supérieure du paravent est portée par la partie inférieure, par l'intermédiaire de moyens de liaison autorisant lesdites manœuvres réversibles entre lesdites positions active et escamotée précitées.

Lesdits moyens de liaison comportent des moyens pour la manœuvre en pivotement de ladite partie supérieure par rapport à ladite partie inférieure, cela autour d'au moins un axe horizontal.
De préférence, la partie supérieure est montée à l'extrémité de deux montants latéraux solidaires de la partie inférieure, lesquels montants sont décalés vers l'arrière par rapport au plan de la paroi frontale de la partie inférieure.

Toujours selon une autre caractéristique, la paroi frontale de la partie inférieure comporte des bordures latérales qui se prolongent, du côté de sa face arrière, par des retours latéraux en matériau radioprotecteur.
De préférence, ces retours latéraux comportent chacun un volet supérieur mobile autour d'une articulation verticale ménagée le long de la bordure latérale de la paroi frontale de la partie inférieure ; et ces volets supérieurs sont mobiles entre une position fermée dans laquelle ils se situent dans le plan ou sensiblement dans le plan desdits retours latéraux, et une position ouverte dans laquelle ils sont aptes à se situer dans le plan ou sensiblement dans le plan de la paroi frontale de la partie inférieure.
Les montants latéraux du paravent sont alors avantageusement ménagés le long de la bordure arrière des retours latéraux de la partie inférieure.

Encore selon une autre particularité, la paroi frontale de la partie supérieure comporte un panneau supérieur réalisé en matériau radioprotecteur transparent qui, une fois en position active, est incliné vers l'avant par rapport à la paroi frontale de la partie inférieure, formant surplomb, pour permettre à l'opérateur de se rapprocher de la zone d'intervention.

De préférence, le panneau supérieur de la partie supérieure est prolongé vers l'arrière par un panneau de couverture réalisé en matériau radioprotecteur (de préférence transparent).

D'autre part, les bordures latérales de ce panneau supérieur, et éventuellement celles du panneau de couverture, se prolongent, vers l'arrière, par des retours latéraux en matériau radioprotecteur.

Alors, l'extrémité inférieure des retours latéraux de la partie supérieure comprennent un rideau souple en matériau radioprotecteur, de manière à réaliser des passages latéraux pour les bras de l'opérateur, dans le prolongement du passage précité ménagé entre les parois frontales des parties inférieure et supérieure du paravent.

De plus, les retours latéraux de la partie supérieure du paravent comportent avantageusement chacun un volet inférieur mobile autour d'une articulation, lesquels volets portent lesdits rideaux souples et sont mobiles entre une position fermée dans laquelle ils se situent dans le plan ou sensiblement dans le plan desdits retours latéraux, et une position ouverte dans laquelle ils sont déployés vers l'extérieur.

Selon encore une autre disposition, la partie supérieure du paravent est solidaire de la partie inférieure par l'intermédiaire de bras latéraux articulés autour d'un premier axe d'articulation horizontal, formant pivot, situé au niveau de l'extrémité supérieure des montants latéraux, lesquels bras latéraux sont solidaires des retours latéraux de ladite partie supérieure, par l'intermédiaire d'un second axe d'articulation horizontal, formant pivot, situé au-dessus dudit premier axe d'articulation, lesquelles articulations sont associées à des moyens de contrôle des mouvements de pivotement, du type vérin(s) par exemple.

De préférence, la paroi frontale de la partie inférieure consiste en un panneau rigide réalisé en matériau radioprotecteur, dans lequel est ménagée une ouverture obturée par un rideau souple en forme d'une pluralité de bandes ou lanières verticales juxtaposées, réalisées en matériau radioprotecteur, permettant le passage d'équipements ou de parties d'équipements au travers de ladite ouverture.

La partie inférieure du paravent est d'autre part avantageusement constituée de deux parties complémentaires coulissantes verticalement l'une par rapport à l'autre, et qui se recouvrent partiellement, pour permettre une variation de sa hauteur, lesquelles deux parties sont assemblées entre elles par des moyens en forme de guides ou glissières associées à un ou plusieurs actionneurs permettant d'adapter la position respective desdites deux parties constitutives.

L'invention concerne également un équipement en forme de housse stérile destiné à recouvrir au moins une partie de la surface d'un paravent tel que défini ci-dessus, cet équipement comprenant :
- une poche souple adaptée pour venir recouvrir l'extrémité inférieure de la paroi frontale de la partie supérieure et munie de moyens pour sa fixation sur ladite paroi frontale,
- deux poches souples adaptées pour venir recouvrir les volets inférieurs mobiles des retours latéraux de la partie supérieure, munies de moyens pour leur fixation sur ledit volet mobile associé,
- une poche souple adaptée pour venir recouvrir l'extrémité supérieure de la paroi frontale de la partie inférieure et les volets supérieurs des retours latéraux associés, munie de moyens pour sa fixation sur ladite paroi frontale et lesdits volets, et
- deux structures pour l'habillage des montants latéraux, munies de moyens pour leur fixation sur lesdits montants latéraux.

D'autres caractéristiques et avantages apparaîtront à la lecture de la description suivante d'une forme de réalisation particulière, donnée uniquement à titre d'exemple et représentée sur les dessins annexés dans lesquels :
- la figure 1 représente un paravent radioprotecteur conforme à l'invention, vu en perspective de ¾ avant, et en position active de radioprotection ;
- la figure 2 est une vue en coupe verticale du paravent de la figure 1 ;
- la figure 3 montre le paravent des figures 1 et 2, vu en perspective de ¾ avant, mais ici avec sa partie supérieure en position partiellement escamotée ;
- la figure 4 est une vue en coupe verticale du paravent de la figure 3 ;
- la figure 5 est encore une vue du même paravent radioprotecteur, avec sa partie supérieure en position complètement escamotée ;
- la figure 6 est une vue en coupe verticale du paravent de la figure 5 ;
- la figure 7 est une vue du paravent avec sa partie supérieure en position partiellement escamotée, et avec ses volets latéraux en position ouverte, illustrant la pose d'un équipement en forme de housse stérile ;
- la figure 8 est une vue de face du paravent selon les figures 1 à 7, ici en position active de radioprotection et avec sa partie inférieure déployée, pour augmenter sa hauteur.

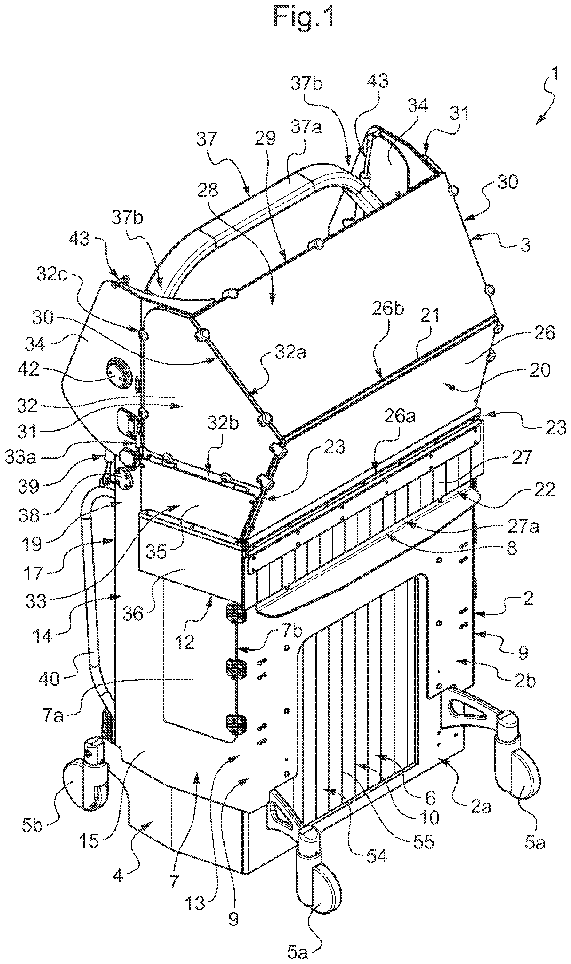

Le paravent radioprotecteur 1 représenté sur les figures 1 à 6 comprend une partie inférieure 2 et une partie supérieure 3 qui sont mobiles l'une par rapport à l'autre, ici par rotation/pivotement de la partie supérieure 3 par rapport à la partie inférieure 2, de manière à permettre sa conformation en différentes configurations détaillées plus loin.

La partie inférieure 2 comporte un piètement ou embase 4, muni de roues 5a, 5b d'appui au sol, à partir duquel s'étend vers le haut une paroi frontale 6 en matériau radioprotecteur (par exemple, pour partie, en acier massif équivalence plomb 2 mm, et pour une autre partie, en matière plastique souple chargée de particules métalliques radio-atténuatrices), prolongée vers l'arrière par deux retours latéraux 7 également en matériau radioprotecteur (par exemple en acier massif équivalence plomb 1 mm).

La paroi frontale 6 et les retours latéraux 7 s'étendent dans un plan vertical ou sensiblement vertical ; ils forment ensemble une structure à section horizontale en U. La paroi frontale 6 est délimitée par une bordure supérieure 8, deux bordures latérales 9, une face avant 10 et une face arrière 11.
La bordure supérieure 8 est horizontale et les bordures latérales 9 sont verticales.

Dans une variante de réalisation, le plan de cette paroi frontale 6 peut être légèrement incliné vers l'avant ou vers l'arrière par rapport à la verticale.

Les deux retours latéraux 7 s'étendent à partir des bordures latérales 9 de la paroi frontale 6, ceci vers l'arrière, c'est-à-dire du côté de la face arrière 11 de cette paroi frontale 6, et à l'équerre ou, ici, de manière légèrement évasée vers l'extérieur.
Ils comportent chacun - une bordure supérieure 12 qui prolonge vers l'arrière la bordure supérieure 8 horizontale de la paroi frontale 6, - une bordure avant 13 raccordée à l'une des bordures latérales 9 de la paroi frontale 6, - une bordure arrière 14, - une face avant 15 (orientée côté extérieur du paravent), et - une face arrière 16 (orientée côté intérieur du paravent).

Ces retours 7 comportent chacun un volet supérieur 7a qui est mobile autour d'une articulation verticale 7b ménagée sur la bordure latérale 9 en regard de la paroi frontale 6 ; chaque volet mobile 7a s'étend sur une partie de la hauteur des retours 7 (par exemple environ la moitié de cette hauteur) et sur tout ou partie de leur largeur. Leur bordure supérieure définit la bordure supérieure 12 des retours 7.

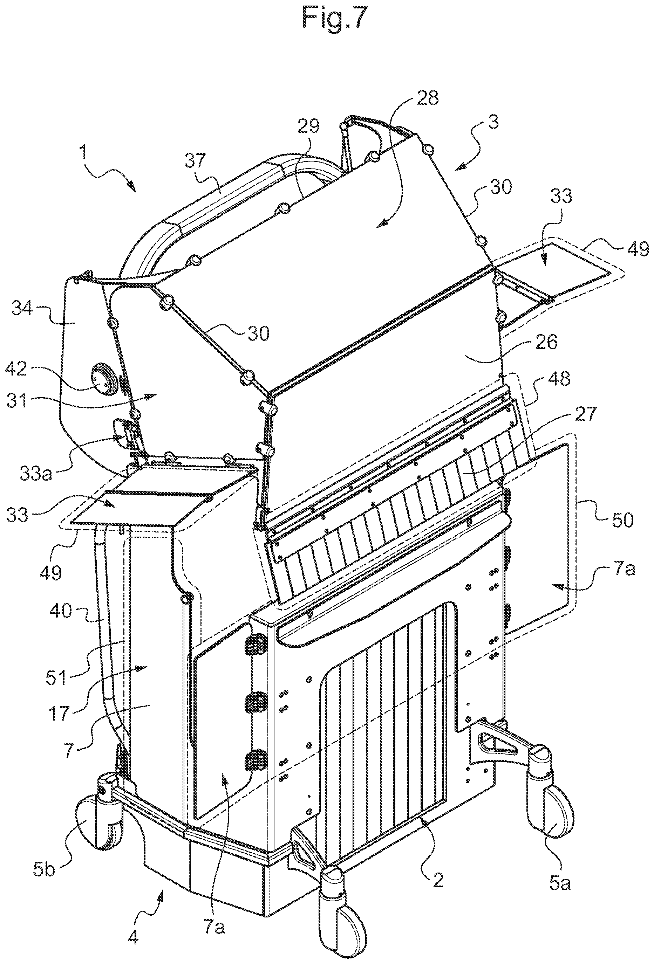

De la sorte ces volets mobiles 7a peuvent occuper une position fermée, telle qu'illustrée sur les figures 1 à 6, dans laquelle ils se situent dans le plan du reste des retours 7 ; ils sont alors verrouillés en position par tout moyen approprié, par exemple un loquet amovible, un aimant, et/ou en utilisant des charnières à cliquet au niveau de leur articulation sur les extrémités de la paroi frontale 6.
Ces volets mobiles 7a peuvent aussi occuper une position ouverte, dans le plan ou sensiblement dans le plan de la paroi frontale 6, comme illustré sur la figure 7 ; alors leur bordure supérieure 12 se situe dans l'alignement de la bordure supérieure 8 de la paroi frontale 6.

Le piètement 4 du paravent 1 comporte - deux roues avant 5a qui s'étendent à partir de la face avant 10 de la paroi frontale 6, sur les côtés et dans l'encombrement de cette dernière, et - deux roues arrière 5b qui s'étendent dans le prolongement des retours latéraux 7.

Deux montants latéraux 17 s'étendent verticalement le long des bordures arrière 14 des retours latéraux 7.
Ces montants 17 peuvent faire partie intégrante des retours latéraux 7, ou bien ils peuvent consister en des éléments rapportés indépendants. Ils sont décalés vers l'arrière par rapport au plan de la paroi frontale 6 et leur extrémité supérieure 19 s'étend au-dessus du niveau des bordures supérieures 12 des retours latéraux 7 et de la bordure supérieure 8 de la paroi frontale 6.

La partie supérieure 3 du paravent 1 consiste en un capot ou caisson monté pivotant sur la partie inférieure 2, et plus particulièrement au niveau de l'extrémité supérieure 19 des montants latéraux 17.

Cette partie supérieure 3 comporte une paroi frontale 20 en matériau radioprotecteur, délimitée par une bordure supérieure 21, une bordure inférieure 22 et deux bordures latérales 23. Elle comporte aussi une face avant 24 et une face arrière 25.

Cette paroi frontale 20 de la partie supérieure 3 comporte un panneau supérieur 26 réalisé en matériau radioprotecteur transparent (par exemple en verre au plomb feuilleté équivalence plomb 2 mm), de forme générale rectangulaire, prolongé vers le bas par un rideau souple 27, formé d'une pluralité de bandes ou lanières verticales juxtaposées, réalisées en matériau radioprotecteur (par exemple une matière plastique souple comportant une charge métallique radio-atténuatrice).
Ce rideau souple 27 s'étend sur toute la largeur du panneau supérieur 26 ; son extrémité supérieure est fixée sur la bordure inférieure 26a de ce panneau 26, par tout moyen approprié ; son extrémité inférieure est libre.

Comme on le verra plus loin, ce rideau souple 27 est destiné à constituer un passage pour les bras de l'opérateur (lorsque le paravent est en configuration active de radioprotection), et sa hauteur peut être comprise entre 20 et 30 cm par exemple.

La bordure inférieure 27a du rideau souple 27 constitue la bordure inférieure 22 de la paroi frontale 20 de la partie supérieure 3 du paravent 1 ; cette bordure inférieure 27a - 22 est libre.

La bordure supérieure 26b du panneau supérieur 26 constitue la bordure supérieure 21 de la paroi frontale 20 ; ici, cette bordure supérieure 26b se prolonge vers le haut et vers l'arrière par un panneau de couverture 28 réalisé également en matériau radioprotecteur, de préférence transparent (par exemple en composite chargé de plomb équivalence plomb 0,5 mm).

Ce panneau de couverture 28 comporte une bordure libre arrière 29 et deux bordures latérales 30.
Ces bordures latérales 30 s'étendent dans le prolongement des bordures latérales 23 du panneau supérieur 26.

Le panneau supérieur 26 et le panneau de couverture 28 sont disposés dans des plans qui forment entre eux un angle de l'ordre de 120°.

Les bordures latérales 23 et 30 du panneau supérieur 26 et du panneau de couverture 28 sont prolongées sur les côtés par des retours latéraux 31 réalisés en matériau radioprotecteur.

Ces retours 31 peuvent s'étendre perpendiculairement ou sensiblement perpendiculairement au plan des panneaux 26 et 28, et donc parallèlement ou sensiblement parallèlement l'un à l'autre ; en l'occurrence, ici, ils s'étendent vers l'arrière de manière légèrement évasée l'un par rapport à l'autre.
La distance qui les sépare correspond approximativement à la distance qui sépare les retours latéraux 7 de la partie inférieure 2.

Chaque retour 31 est en fait constitué d'un panneau supérieur 32 en matériau radioprotecteur de préférence transparent (par exemple en composite chargé de plomb équivalence plomb 0,5 mm) - dont la bordure avant 32a est raccordée avec les bordures latérales des panneaux supérieur 26 et de couverture 28, - dont la bordure inférieure 32b, qui s'étend parallèlement ou sensiblement parallèlement à la bordure inférieure 26a du panneau 26 et qui est disposée légèrement au-dessus de cette dernière, est prolongée vers le bas par un volet 33 en matériau radioprotecteur, et - dont la bordure arrière 32c est prolongée par une platine 34, également en matériau radioprotecteur (par exemple en acier massif équivalence plomb 1 mm).

Les volets 33 sont montés pivotants autour de la bordure 32b qui les rattache au panneau supérieur 32, au moyen de charnières. Ils sont constitués d'une plaque/embase 35 en matériau radioprotecteur (par exemple en acier massif équivalence plomb 0,75 mm), prolongée vers le bas par un rideau souple 36 également en matériau radioprotecteur (par exemple en matière plastique souple comportant une charge métallique radio-atténuatrice). La bordure inférieure de la plaque/embase 35 s'étend au niveau de la bordure inférieure 26a du panneau supérieur 26. Les deux rideaux souples 36 prolongent sur les côtés, le rideau souple 27 de la paroi frontale 20.

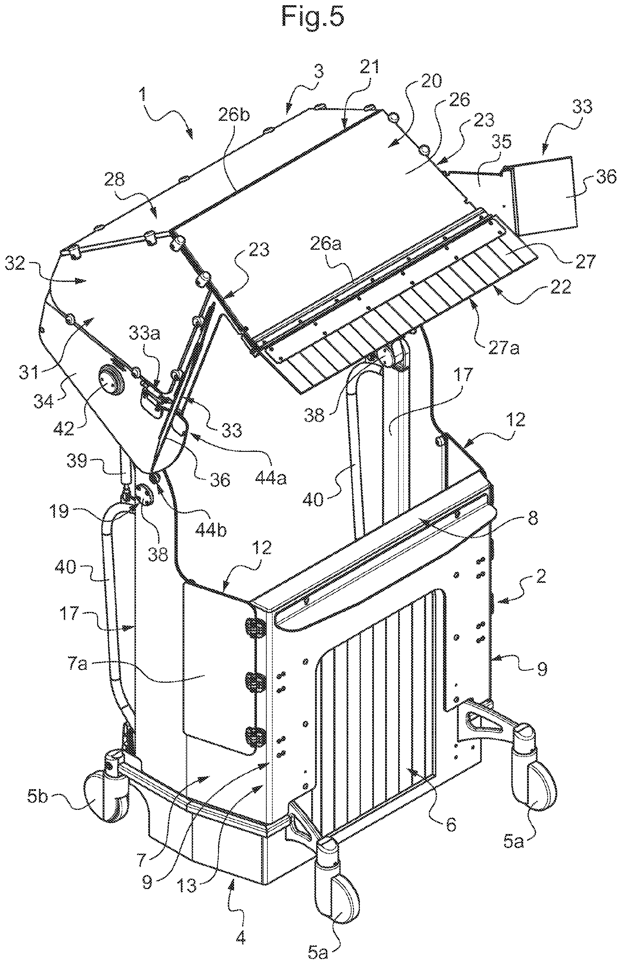

Lorsque les volets 33 sont placés dans le plan de leur panneau support supérieur 32, les rideaux souples 36 sont positionnés dans la continuité du rideau souple avant 27 et ils s'étendent vers l'arrière, à l'équerre ou de manière légèrement évasée vers l'extérieur.
La hauteur des rideaux souples 36 correspond à celle du rideau souple avant 27.
Les deux volets latéraux 33 sont mobiles en pivotement autour de la charnière ménagée sur la bordure 32b précitée, cela entre une position fermée, dans laquelle ils se situent dans le plan des panneaux supérieurs 32 (figures 1 à 4) et une position ouverte dans laquelle ils sont écartés extérieurement (figures 5 à 7).
Leur manœuvre peut s'effectuer manuellement et/ou de manière automatique par un système de câble(s) associé au pivotement de la partie supérieure 3 du paravent, comme détaillé plus loin. L'articulation correspondante est associée à des moyens de contrôle du mouvement, constitués d'un vérin 33a, par exemple du type vérin à gaz, interposé entre la plaque/embase 35 et la platine 34 juxtaposée. Le vérin 33a a pour fonction de maintenir le volet 33 dans la position souhaitée, en particulier pour la pose des housses stériles.

Les deux platines 34 sont raccordées par un arceau porteur 37 formé d'une traverse supérieure 37a dont les extrémités sont prolongées vers l'arrière par deux bras latéraux 37b.

La traverse 37a s'étend horizontalement, approximativement dans le plan de l'extrémité supérieure des retours latéraux 31 et en retrait vers l'arrière par rapport à la bordure arrière 29 du panneau de couverture 28.

L'extrémité inférieure des bras 37b est assemblée avec l'extrémité supérieure 19 des montants latéraux 17 de la partie inférieure 2 du paravent par l'intermédiaire d'un premier axe d'articulation horizontal 38, formant pivot, dont le mouvement de pivotement est contrôlé par deux vérins latéraux 39, par exemple du type vérin à gaz.

Chacun des vérins 39 est interposé entre une première chape fixée sur l'un des bras latéraux 37b et une seconde chape fixée sur le montant latéral 17 en correspondance (ici, cette seconde chape est portée par l'extrémité supérieure d'un arceau 40 adapté pour la manœuvre du paravent et dont chaque montant 17 est équipé).

Les fins de course des deux vérins 39 définissent le degré de liberté en rotation de la partie supérieure 3 par rapport à la partie inférieure 2, autour de l'axe 38 ; et ces deux vérins 39 permettent d'amortir le mouvement correspondant, dans les deux sens.

D'autre part, les bras latéraux 37b sont solidaires des retours latéraux 31 (et en particulier de leurs platines 34) par l'intermédiaire d'un second axe d'articulation horizontal 42, formant pivot, dont le mouvement est contrôlé par deux vérins latéraux 43, par exemple également du type vérin à gaz.

Chacun de ces vérins 43 est interposé entre une première chape fixée sur l'un des bras latéraux 37b et une seconde chape fixée sur la platine 34 en correspondance.

Les fins de course des deux vérins 43 définissent le degré de liberté en rotation de la partie supérieure 3 par rapport à l'arceau 37 (et donc aussi par rapport à la partie inférieure 2), autour de l'axe 42. Ces deux vérins 43 permettent d'amortir le mouvement correspondant, dans les deux sens.

De préférence, la manœuvre en pivotement autour de l'axe 42 est rendue possible uniquement lorsque l'arceau 37 est en basculement arrière maximal par rapport aux montants latéraux 17, ceci par un système de butées d'accroche 44a, 44b ; dans l'autre sens, le retour en position fermée s'effectue tout d'abord par le basculement autour de l'axe 42, puis autour de l'axe 38, toujours grâce au système de butées d'accroche 44a, 44b.

Le mouvement de basculement autour des axes 38 et 42 est obtenu par une manœuvre manuelle de l'opérateur sur la partie supérieure 3, au moyen de poignées de manœuvre 45 prévues au niveau de la bordure inférieure du panneau supérieur 26, côté face arrière de celui-ci, pour être accessibles depuis l'intérieur du paravent 1.

Une telle structure autorise une mobilité de la partie supérieure 3 par rapport à la partie inférieure 2 du paravent, par pivotement autour des liaisons pivots 38 et 42 précitées.

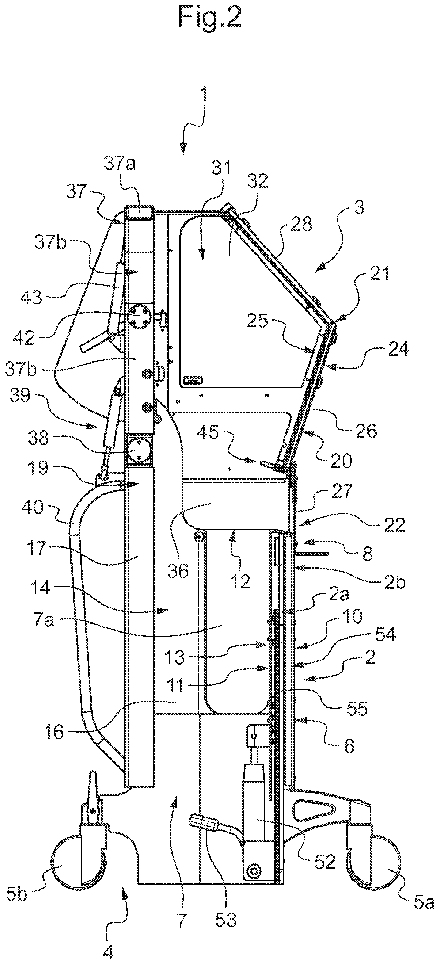

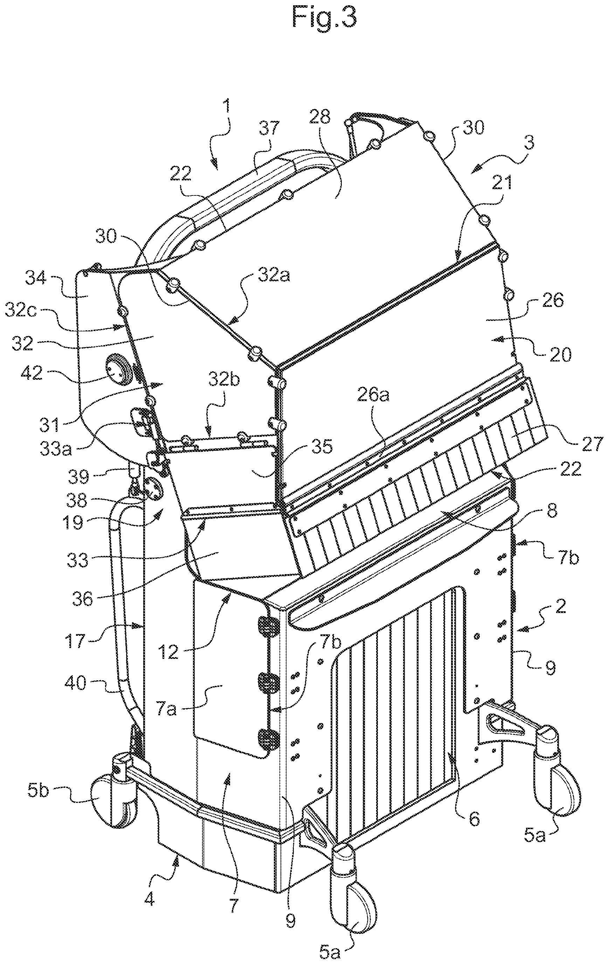

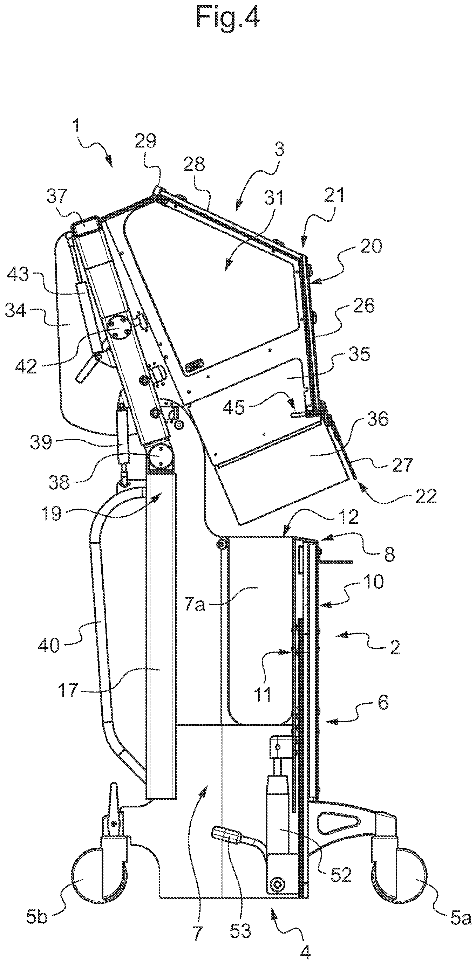

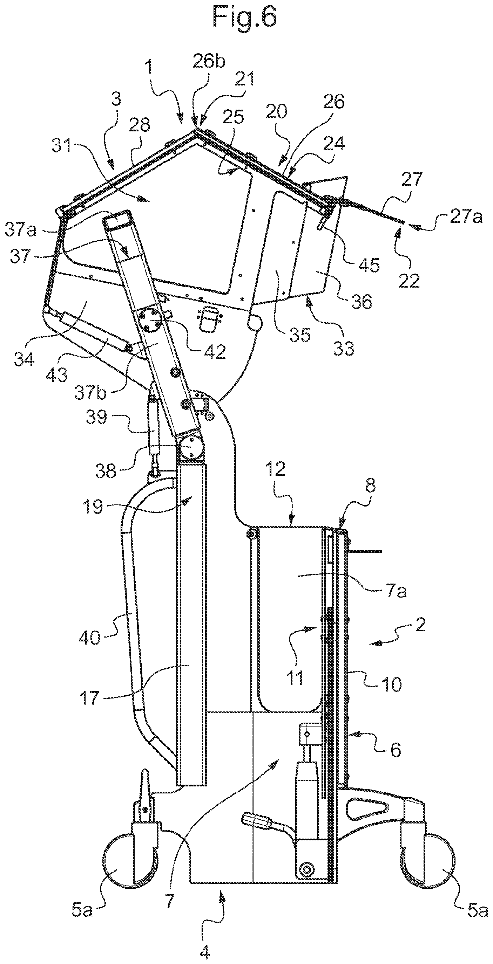

Les parois frontales 20 et 6 des parties supérieure 3 et inférieure 2, ainsi que les retours latéraux 31 et 7 qui leur sont associés sont ainsi séparables de manière réversible, et il est possible de conformer le paravent selon différentes configurations, en particulier :
- une configuration dite « active », illustrée sur les figures 1 et 2, dans laquelle la partie supérieure 3 est abaissée vers l'avant en direction de la partie inférieure 2, de telle sorte que leurs parois frontales 6 et 20 viennent dans le prolongement l'une de l'autre (tout comme leurs retours associés 7 et 31) pour assurer une protection efficace contre les rayonnements ionisants,
- une configuration dite « escamotée » partielle, illustrée sur les figures 3 et 4, dans laquelle la partie supérieure 3 est basculée vers le haut et légèrement vers l'arrière par un mouvement de pivotement autour de l'axe 38, de manière à séparer lesdites parois frontales inférieure 6 et supérieure 20, avec leurs retours associés, et libérer ainsi un petit espace au-dessus de la partie inférieure 3, et
- une configuration dite « escamotée complète » illustrée sur les figures 5 et 6 dans laquelle la partie supérieure 3 est basculée éventuellement un peu vers le haut mais aussi largement vers l'arrière par un mouvement de pivotement complémentaire autour de l'axe 42, offrant à l'opérateur une liberté de mouvement importante par-dessus la partie inférieure 3, par exemple en direction d'un patient alité devant le paravent ; l'espace libéré correspondant est frontal et également latéral, du fait du déport vers l'arrière des montants latéraux 17 qui portent la partie supérieure 3 du paravent.

De manière avantageuse, un système de commande par câbles assure l'écartement vers l'extérieur des deux volets 33 lors du pivotement arrière de la partie supérieure 3 autour de l'axe horizontal supérieur 42. Cette particularité permet de libérer encore davantage l'espace pour l'opérateur, en particulier sur les côtés, comme illustré sur les figures 5 et 6.
Les câbles correspondants, non visibles sur les figures, raccordent de manière adaptée les bras 37b de l'arceau 37 et les plaques/embases 35, en passant par des renvois adaptés.

L'axe de rotation 42 s'étend au-dessus de l'axe 38 ; il est positionné approximativement à mi-hauteur des bras 37b.

La présence de ces deux axes de rotation 38 et 42, décalés en hauteur, procure à la partie supérieure 3 une cinématique optimale, lors de son basculement vers l'arrière ou vers l'avant, en limitant son niveau maximal d'élévation. Cela permet de limiter l'espace en hauteur nécessaire pour la manœuvre de la partie supérieure 3 du paravent, par exemple pour éviter le contact avec des appareils ou parties d'appareils situés en hauteur.

A titre de variante, la partie supérieure 3 du paravent peut être articulée sur les montants latéraux 17 autour d'un axe horizontal unique, en association avec une capacité de pivotement adaptée.

Selon encore une autre variante de réalisation, on peut aussi envisager de solidariser la partie supérieure 3 du paravent avec les montants latéraux 17 par l'intermédiaire de glissières verticales, en association avec un ou plusieurs actionneurs adaptés, pour permettre son mouvement vertical en hauteur, pour obtenir les positions active et escamotée recherchées.

Dans la position active de radioprotection (figure 1 et 2) la paroi frontale 6 de la partie inférieure 2 et la paroi frontale 20 de la partie supérieure 3 sont disposées dans le prolongement l'une de l'autre, tout comme leurs retours latéraux 7 et 31. Les bras 37b sont dans l'alignement des montants latéraux 17.

L'extrémité inférieure 27a du rideau souple 27 arrive au niveau de la bordure supérieure 8 de la paroi frontale 6 de la partie inférieure 2, ou vient en léger recouvrement de cette dernière.
Et de la même manière, les extrémités inférieures des rideaux souples latéraux 33 arrivent au niveau de la bordure supérieure 12 des retours latéraux 7 de la partie inférieure 2, ou viennent en léger recouvrement de ces derniers.

Les rideaux souples 27 et 33 en matériau radioprotecteur, présents au niveau de la zone de juxtaposition entre les parties inférieure 2 et supérieure 3 assurent ainsi un passage pour les bras de l'opérateur qui va pouvoir accéder au patient et/ou à certains équipements présent(s) juste devant le paravent, cela tout en restant efficacement protégé contre les radiations.

Ce passage de bras est à section horizontale en U, sans discontinuité entre la face avant et les côtés, ce qui assure un confort d'intervention optimal pour l'opérateur. Sa hauteur, par exemple de l'ordre de 20 à 30 cm, est adaptée pour optimiser ce confort, tout en étant minimisée de manière à conserver une radioprotection efficace.

Le passage de bras correspondant est ici ménagé au niveau de la bordure inférieure de la paroi frontale supérieure 20 et de ses retours latéraux 31 ; les rideaux souples 27 et 36 s'étendent verticalement par simple gravité.
Dans une variante de réalisation, ces rideaux souples peuvent être prévus au niveau de la bordure supérieure de la paroi frontale et des retours latéraux de la partie inférieure du paravent ; ces rideaux souples seront alors maintenus en position verticale par tout moyen approprié.

Comme on peut le voir sur la figure 1, dans cette position active de radioprotection, le panneau supérieur 26 en matériau radioprotecteur transparent est incliné vers l'avant par rapport à la paroi frontale verticale 6 de la partie inférieure 2 du paravent. Ce panneau transparent 26 forme ainsi surplomb et permet à l'opérateur de se rapprocher de la zone d'intervention située devant le paravent.
L'angle de surplomb correspondant est avantageusement compris entre 10 et 30° par rapport à la verticale (il est de préférence de l'ordre de 15 à 20° par rapport à la verticale).

Dans cette position active de radioprotection, la tige des vérins 39 est en butée dans l'une de ses positions stables, ici extraite maximale.

Lorsque l'opérateur le souhaite, par exemple en début d'intervention avant la mise en œuvre des moyens pour générer des rayonnements ionisants, ou en cours d'intervention, par exemple pour accéder directement au patient, il peut relever la partie supérieure 3 du paravent comme illustré sur les figures 5 et 6, de manière à créer une ouverture frontale d'accès, au-dessus des bordures supérieures 8 et 12 de la partie inférieure 2.

Pour cela, partant de la position active illustrée sur les figures 1 et 2, l'opérateur commence à basculer la partie supérieure 3 vers le haut, en saisissant les poignées de manœuvre 45.
La partie supérieure 3 pivote alors autour de l'articulation horizontale 38, sous contrôle des vérins 39, pour obtenir la position escamotée intermédiaire illustrée sur les figures 3 et 4.
La tige des vérins 39 atteint son autre position stable (position rentrée) le pivotement correspondant peut être de l'ordre de 18 °.
Comme expliqué auparavant, pendant ce premier pivotement autour de l'axe 38 l'articulation autour de l'axe 42 est verrouillée par le système de butées d'accroche 44a, 44b.

Une fois cette première position intermédiaire atteinte, le jeu de butées d'accroche adapté 44a, 44b libère l'axe 42, et une poussée supplémentaire appliquée vers le haut par l'opérateur sur les poignées de manœuvre 45 entraîne le pivotement de la partie supérieure 3 autour de cet axe 42.
Ce pivotement s'effectue sous le contrôle des vérins 43, jusqu'à ce qu'ils atteignent leur seconde position stable, ici avec leur tige complètement rentrée.
On obtient alors la position escamotée complète illustrée sur les figures 5 et 6.
Ce second pivotement peut être réalisé sur un secteur angulaire de l'ordre de 60°, pour obtenir au total (c'est-à-dire avec le pivotement autour de l'axe 38), un pivotement de l'ordre de 78°.
Comme précisé auparavant, le mouvement de pivotement vers le haut et vers l'arrière autour de l'axe 42 s'accompagne avantageusement de l'écartement extérieur des volets 33 (comme visible sur les figures 5 et 6), par exemple sur un secteur angulaire de l'ordre de 40 à 60°, grâce à un jeu de câbles adapté.

Lorsque l'opérateur souhaite revenir en position active de radioprotection, il tire sur les poignées de manœuvre 45 pour commencer à abaisser la partie supérieure 3, par pivotement autour de l'axe d'articulation 42, jusqu'à ce que les vérins 43 atteignent leur position stable avec leur tige complètement extraite. Ensuite, une poursuite de la manœuvre de traction entraîne le second pivotement autour de l'axe 38, sous contrôle des vérins 39, jusqu'à ce que la tige de ces derniers arrive dans leur position stable extraite, pour obtenir la configuration active du paravent illustré sur les figures 1 et 2.

On comprend donc qu'il est possible d'obtenir les positions active de radioprotection et escamotée complète, de manière très simple et rapide.

Dans le cadre de l'utilisation du paravent 1 dans une ambiance stérile (par exemple pour une intervention chirurgicale), il est prévu de recouvrir au moins une partie de ses différentes faces constitutives, et le paravent est structuré pour permettre une pose simple et rapide de ce système de housse.

Dans le cas présent, on utilise un équipement formé d'une pluralité d'éléments indépendants stériles pour constituer cette housse de couverture. Et la mise en place, ainsi que le retrait de certains de ces éléments sont réalisés en association avec le pivotement des volets 7a et 33 précités, comme illustré sur la figure 7.

Comme on peut le voir sur cette figure 7, la mise en place de cet équipement stérile s'effectue alors que le paravent est en position intermédiaire escamotée (c'est-à-dire avec sa partie supérieure 3 pivotée vers l'arrière autour de l'axe 38 uniquement). En outre, pour cela, les volets 33 des retours 31 sont écartés manuellement vers l'extérieur (positionnés par exemple à l'équerre par rapport au panneau supérieur 32, et ils sont maintenus dans cette position par l'action des vérins associés 33a ; de plus, les volets inférieurs 7a des retours 7 sont ouverts et placés dans le plan ou sensiblement dans le plan de la paroi frontale 6.

Comme illustré en pointillés sur la figure 7, l'équipement stérile utilisé pour assurer la protection du paravent 1 comprend plusieurs éléments dont :
- une poche souple 48, adaptée pour venir recouvrir l'extrémité inférieure de la paroi frontale 20 de la partie supérieure 3. Cette poche souple 48 consiste en deux panneaux parallèles, assemblés sur trois de leurs bordures et munis d'une ouverture pour son positionnement autour du rideau souple 27 et de la partie inférieure du panneau transparent 26.
Cette poche souple 48 peut être réalisée en tout matériau adapté, de préférence transparent ; son ouverture de positionnement peut être bordée par des moyens élastiques pour faciliter sa mise en place ; elle est encore avantageusement munie de moyens pour assurer sa fixation sur le paravent, par exemple un système adhésif et/ou de ventouse(s). - deux poches souples 49, adaptées pour venir recouvrir les volets inférieurs mobiles 33 des retours latéraux 31 de la partie supérieure 3.
Chacune de ces poches 49 est formée de deux volets rectangulaires solidarisés sur trois de leurs bordures, la quatrième bordure constituant une ouverture de positionnement, avantageusement associée à des moyens élastiques.
Chacune de ces poches 49 est réalisée en tout matériau approprié, par exemple non transparent. Leur ouverture peut être bordée par des moyens élastiques pour faciliter leur positionnement ; et chacune de ces poches souples 49 est munie de moyens pour sa fixation sur le volet 33 associé, par exemple des moyens adhésifs ou ventouse(s). - une poche souple 50 adaptée pour venir recouvrir l'extrémité supérieure de la paroi frontale 6 de la partie inférieure 2 et les volets supérieurs 7a des retours latéraux 7.
Là encore, cette poche souple 50 est constituée de deux volets rectangulaires assemblés au niveau de trois de leurs bordures, et dont la quatrième constitue une ouverture de positionnement. Elle est réalisée en tout matériau approprié, par exemple transparent et comporte des moyens pour sa fixation sur le paravent, par exemple du type adhésif ou ventouse(s).
Son ouverture peut être bordée par des moyens élastiques pour faciliter son positionnement.
- deux structures 51 pour l'habillage des montants latéraux 17 (avec éventuellement une partie des retours 7). Chacune de ces structures 51 peut être constituée d'un simple panneau rectangulaire en matériau approprié (par exemple en matériau non tissé), qui est conformé pour venir recouvrir la périphérie des montants 17 (avec éventuellement une partie des retours 7) et qui est maintenu en place par tout moyen de fixation approprié, par exemple des moyens adhésifs.

Une fois les différents éléments 48 à 51 de l'équipement stérile correctement positionnés, les volets 33 et 7a sont replacés manuellement en position fermée.

Le niveau de positionnement en hauteur de la bordure supérieure 8 de la partie inférieure 2 du paravent correspond avantageusement au niveau de l'appui des avant-bras de l'opérateur, pour optimiser son confort d'utilisation.
La hauteur correspondante par rapport au sol peut être de l'ordre de 110 à 130 cm.

La partie inférieure 2 du paravent 1 est de préférence réalisée en deux parties télescopiques 2a et 2b pour autoriser un ajustement de la hauteur du paravent, et aussi du positionnement en hauteur des rideaux souples 27, 33 pour le passage des bras, en particulier en fonction de la taille de l'opérateur.

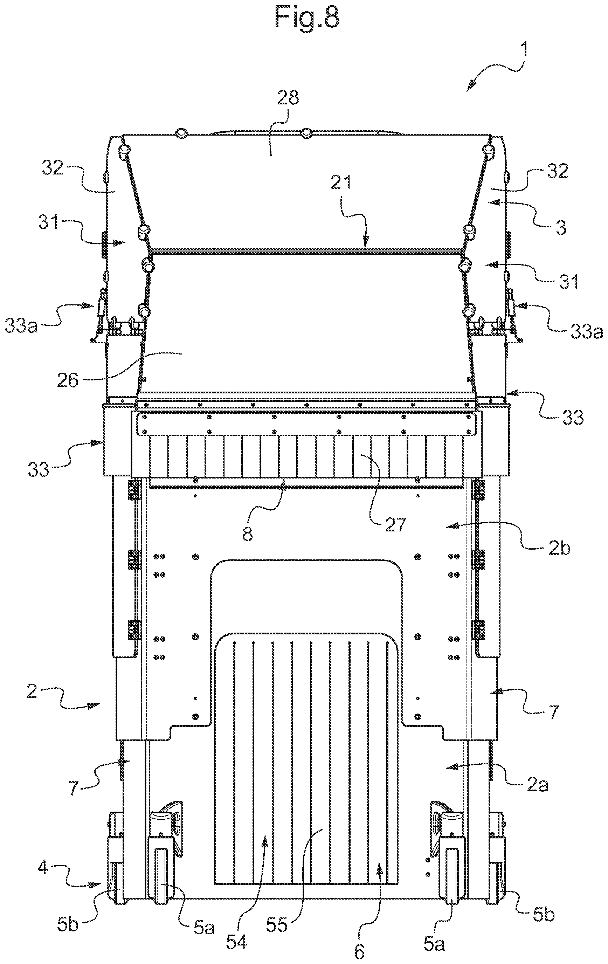

La partie basse 2a est solidaire du piètement 4 et est assemblée avec la partie haute télescopique 2b au moyen de guides ou de glissières adaptées (non visibles sur les figures), associées à des moyens de manœuvre en forme d'actionneur(s) 52.
Par exemple, on peut utiliser un actionneur 52 du type vérin hydraulique, associé à un levier de commande 53 manœuvrable au pied (pédale de commande), apte à gérer un mouvement d'élévation ou d'abaissement du paravent. Les figures 1 à 7 illustrent le paravent 1 en position basse, avec sa partie inférieure 2 en position rétractée complète. La figure 8 est une vue de face du paravent 1, illustré ici en position haute avec sa partie inférieure 2 déployée.

Sur les figures 1 à 8, on remarque que la zone centrale de la paroi frontale 6 de la partie inférieure 2 comporte une ouverture 54 obturée par un rideau souple 55 en forme d'une pluralité de bandes ou de lanières verticales juxtaposées, réalisées en matériau radioprotecteur (par exemple en matière plastique chargée de particules métalliques radio-atténuatrices).

Une telle ouverture 54 associée à un rideau souple 55 permet le passage d'équipements ou de parties d'équipements en son travers (par exemple la tête de l'arceau émetteur de rayons X), tout en conservant une radioprotection efficace.

Par exemple, l'ouverture 54 en question peut être réalisée dans un panneau rigide en matériau radioprotecteur constitutif de la partie basse 2a de la partie inférieure 2, sur la bordure supérieure de laquelle sont fixées les bandes ou lanières souples en matériau radioprotecteur ; et la partie haute télescopique 2b de la partie inférieure 2 comporte alors une ouverture en regard, adaptée pour ne pas gêner le passage au travers dudit rideau souple.

## Revendications

1. Paravent (1) en matériau radioprotecteur pour la protection d'un opérateur à l'encontre des rayonnements ionisants, lequel paravent (1) comprend :
(i) une partie inférieure (2) qui comporte un piètement (4), et au moins une paroi frontale (6) en matériau radioprotecteur, laquelle paroi frontale (6) s'étend dans un plan vertical ou sensiblement vertical et comporte une bordure supérieure (8), une face avant (10) et une face arrière (11),
(ii) une partie supérieure (3) comportant au moins une paroi frontale (20) en matériau radioprotecteur, dont une partie (26) au moins est transparente pour autoriser un accès visuel en son travers, laquelle paroi frontale (20) comporte une face avant (24), une face arrière (25) et une bordure inférieure (22), et
(iii) au moins un passage (27) pour les bras de l'opérateur,
ladite paroi frontale (6) de la partie inférieure (2) et ladite paroi frontale (20) de la partie supérieure (3) étant disposées dans le prolongement l'une de l'autre, par juxtaposition de leurs bordures supérieure (8) et inférieure (22) respectives, occupant ainsi une position active assurant la protection recherchée,
lesdites parois frontales (6, 20) de la partie supérieure (3) et de la partie inférieure (2) étant séparables l'une de l'autre,
**caractérisé en ce que** ladite partie supérieure (3) est portée par ladite partie inférieure (2), par l'intermédiaire de moyens de liaison (17, 38, 37, 42) autorisant sa manœuvre réversible entre ladite position active et une position escamotée dans laquelle la bordure inférieure (22) de ladite paroi frontale (20) de ladite partie supérieure (3) est écartée de la bordure supérieure (8) de ladite paroi frontale (6) de ladite partie inférieure (2), pour libérer un espace au-dessus de ladite partie inférieure (2),
lesquels moyens de liaison (17, 38, 37, 42) comportent des moyens pour la manœuvre en pivotement (38, 42) de ladite partie supérieure (3) par rapport à ladite partie inférieure (2), cela autour d'au moins un axe horizontal (38, 42).

2. Paravent (1) selon la revendication 1, **caractérisé en ce que** ledit passage (27) pour les bras de l'opérateur est ménagé au niveau de la zone de juxtaposition entre lesdites parties inférieure (2) et supérieure (3), lorsque ces dernières sont en position juxtaposée active.

3. Paravent (1) selon la revendication 2, **caractérisé en ce que** ledit passage pour les bras de l'opérateur est ménagé au niveau de la bordure inférieure de la paroi frontale (20) de la partie supérieure (3) et consiste en un rideau souple (27) en forme d'une pluralité de bandes ou lanières verticales juxtaposées, réalisées en matériau radioprotecteur, et dont l'extrémité supérieure est fixée sur un panneau supérieur (26) réalisé en matériau radioprotecteur.

4. Paravent (1) selon l'une quelconque des revendications 1 à 3, **caractérisé en ce que** la partie supérieure (3) est montée à l'extrémité de deux montants latéraux (17) solidaires de la partie inférieure (2), lesquels montants (17) sont décalés vers l'arrière par rapport au plan de la paroi frontale (6) de ladite partie inférieure (2).

5. Paravent (1) selon l'une quelconque des revendications 1 à 4, **caractérisé en ce que** ladite paroi frontale (6) de la partie inférieure (2) comporte des bordures latérales (9) qui se prolongent, du côté de sa face arrière (11), par des retours latéraux (7) en matériau radioprotecteur, lesdits retours latéraux (7) comportant chacun un volet supérieur (7a) mobile autour d'une articulation verticale (7b) ménagée le long de la bordure latérale (9) en regard de la paroi frontale (6) de la partie inférieure (2), lesquels volets supérieurs (7a) sont mobiles entre une position fermée dans laquelle ils se situent dans le plan ou sensiblement dans le plan desdits retours latéraux (7), et une position ouverte dans laquelle ils sont aptes à se situer dans le plan ou sensiblement dans le plan de ladite paroi frontale (6) de la partie inférieure (2).

6. Paravent (1) selon les revendications 4 et 5 prises en combinaison, **caractérisé en ce que** lesdits montants latéraux (17) sont ménagés le long de la bordure arrière (14) des retours latéraux (7) de la partie inférieure (2).

7. Paravent (1) selon l'une quelconque des revendications 1 à 6, **caractérisé en ce que** la paroi frontale (20) de la partie supérieure (3) comporte un panneau supérieur (26) réalisé en matériau radioprotecteur transparent qui, une fois en position active, est incliné vers l'avant par rapport à la paroi frontale (6) de la partie inférieure (2), formant surplomb, pour permettre à l'opérateur de se rapprocher de la zone d'intervention.

8. Paravent (1) selon la revendication 7, **caractérisé en ce que** ledit panneau supérieur (26) de la partie supérieure (3) est prolongé vers l'arrière par un panneau de couverture (28) réalisé en matériau radioprotecteur, les bordures latérales (23, 30) dudit panneau supérieur (26) et dudit panneau de couverture (28), se prolongeant, vers l'arrière, par des retours latéraux (31) en matériau radioprotecteur.

9. Paravent (1) selon les revendications 3 et 8, prises en combinaison, **caractérisé en ce que** l'extrémité inférieure des retours latéraux (31) de ladite partie supérieure (3) comprennent un rideau souple (36) en matériau radioprotecteur, de manière à réaliser des passages latéraux pour les bras de l'opérateur, dans le prolongement dudit passage (27) ménagé entre lesdites parois frontales (6 et 20) des parties inférieure (2) et supérieure (3).

10. Paravent (1) selon l'une quelconque des revendications 8 ou 9, **caractérisé en ce que** lesdits retours latéraux (31) de ladite partie supérieure (3) comportent chacun un volet inférieur (33) mobile autour d'une articulation (32b), lesquels volets (33) comportent lesdits rideaux souples (36) et sont mobiles entre une position fermée dans laquelle ils se situent dans le plan ou sensiblement dans le plan desdits retours latéraux (31), et une position ouverte dans laquelle ils sont déployés vers l'extérieur.

11. Paravent (1) selon les revendications 1, 4 et 8, prises en combinaison, **caractérisé en ce que** la partie supérieure (3) est solidaire de la partie inférieure (2) par l'intermédiaire de bras latéraux (37b) articulés autour d'un premier axe d'articulation horizontal (38), formant pivot, situé au niveau de l'extrémité supérieure (19) des montants latéraux (17), lesquels bras latéraux (37b) sont solidaires des retours latéraux (31) de ladite partie supérieure (3), par l'intermédiaire d'un second axe d'articulation horizontal (42), formant pivot, situé au-dessus dudit premier axe d'articulation (38), lesquelles articulations (38, 42) sont associées à des moyens de contrôle des mouvements de pivotement (39, 43) du type vérins par exemple.

12. Paravent (1) selon l'une quelconque des revendications 1 à 11, **caractérisé en ce que** la paroi frontale (6) de la partie inférieure (2) consiste en un panneau rigide réalisé en matériau radioprotecteur (2a), dans lequel est ménagée une ouverture (54) obturée par un rideau souple (55) en forme d'une pluralité de bandes ou lanières verticales juxtaposées, réalisées en matériau radioprotecteur, permettant le passage d'équipements ou de parties d'équipement au travers de ladite ouverture (54).

13. Paravent (1) selon l'une quelconque des revendications 1 à 12, **caractérisé en ce que** la partie inférieure est constituée de deux parties complémentaires (2a, 2b) coulissantes verticalement l'une par rapport à l'autre, et qui se recouvrent partiellement, pour permettre une variation de sa hauteur, lesquelles deux parties (2a, 2b) sont assemblées entre elles par des moyens en forme de guides ou glissières associées à un ou plusieurs actionneur(s) (52) permettant d'adapter la position respective desdites deux parties constitutives.

14. Equipement en forme de housse stérile destiné à recouvrir au moins une partie de la hauteur d'un paravent (1) selon les revendications 5 et 10 prises en combinaison, l'équipement comprend :
- une poche souple (48) adaptée pour venir recouvrir l'extrémité inférieure de la paroi frontale (20) de la partie supérieure (3) et munie de moyens pour sa fixation sur ladite paroi frontale (20),
- deux poches souples (49) adaptées pour venir recouvrir les volets inférieurs mobiles (33) des retours latéraux (31) de la partie supérieure (3), munies de moyens pour leur fixation sur ledit volet mobile associé (33),
- une poche souple (50) adaptée pour venir recouvrir l'extrémité supérieure de la paroi frontale (6) de la partie inférieure (2) et les volets supérieurs (7a) des retours latéraux associés (7), munie de moyens pour sa fixation sur ladite paroi frontale (6) et lesdits volets (7a), et
- deux structures (51) pour l'habillage des montants latéraux (17), avec éventuellement une partie des retours (7), munies de moyens pour leur fixation sur lesdits montants latéraux (07) et éventuellement les retours (7).

## Patentansprüche

1. Schirm (1) aus einem Strahlenschutzmaterial zum Schutz eines Bedieners vor ionisierender Strahlung, wobei der Schirm (1)
(i) einen unteren Teil (2), der ein Fußgestell (4) aufweist, und wenigstens eine Frontwandung (6) aus Strahlenschutzmaterial, wobei sich die Frontwandung (6) in einer senkrechten oder im Wesentlichen senkrechten Ebene erstreckt und einen oberen Rand (8), eine Vorderseite (10) und eine Rückseite (11) aufweist,
(ii) einen oberen Teil (3), der wenigstens eine Frontwandung (20) aus Strahlenschutzmaterial aufweist, von der wenigstens ein Teil (26) durchsichtig ist, um einen visuellen Zugang durch ihn hindurch zu ermöglichen, wobei die Frontwandung (20) eine Vorderseite (24), eine Rückseite (25) und einen unteren Rand (22) aufweist, und
(iii) wenigstens einen Durchgang (27) für die Arme des Bedieners aufweist,
wobei die Frontwandung (6) des unteren Teils (2) und die Frontwandung (20) des oberen Teils (3) unter Nebeneinanderstellung ihres jeweiligen oberen (8) beziehungsweise unteren (22) Rands in Verlängerung zueinander angeordnet sind und so eine aktive Stellung einnehmen, die den gewünschten Schutz sicherstellt, wobei die Frontwandungen (6, 20) des oberen Teils (3) und des unteren Teils (2) voneinander trennbar sind,
**dadurch gekennzeichnet, daß** der obere Teil (3) vom unteren Teil (2) mittels Verbindungsmitteln (17, 38, 37, 42) getragen wird, die eine umkehrbare Betätigung zwischen der aktiven Stellung und einer zusammengeklappten Stellung ermöglichen, in der der untere Rand (22) der Frontwandung (20) des oberen Teils (3) vom oberen Rand (8) der Frontwandung (6) des unteren Teils (2) beabstandet ist, um einen Raum oberhalb des unteren Teils (2) freizumachen,
wobei die Verbindungsmittel (17, 38, 37, 42) Mittel (38, 42) zur schwenkenden Betätigung des oberen Teils (3) gegenüber dem unteren Teil (2), und zwar um wenigstens eine horizontale Achse (38, 42), aufweisen.

2. Schirm (1) gemäß Anspruch 1, **dadurch gekennzeichnet, daß** der Durchgang (27) für die Arme des Bedieners im Bereich des Gebiets der Nebeneinanderstellung zwischen dem unteren (2) und dem oberen (3) Teil eingerichtet ist, wenn letztere in der aktiven, nebeneinandergestellten Stellung sind.

3. Schirm (1) gemäß Anspruch 2, **dadurch gekennzeichnet, daß** der Durchgang für die Arme des Bedieners im Bereich des unteren Rands der Frontwandung (20) des oberen Teils (3) eingerichtet ist und aus einem flexiblen Vorhang (27) in Form einer Anzahl senkrechter, nebeneinander angeordneter, aus Strahlenschutzmaterial gefertigter Bänder oder Streifen besteht, deren oberes Ende an einer aus Strahlenschutzmaterial gefertigten oberen Platte (26) befestigt ist.

4. Schirm (1) gemäß einem der Ansprüche 1 bis 3, **dadurch gekennzeichnet, daß** der obere Teil (3) am Ende zweier mit dem unteren Teil (2) fest verbundener seitlicher Stützen (17) angebracht ist, wobei die Stützen (17) gegenüber der Ebene der Frontwandung (6) des unteren Teils (2) nach hinten versetzt sind.

5. Schirm (1) gemäß einem der Ansprüche 1 bis 4, **dadurch gekennzeichnet, daß** die Frontwandung (6) des unteren Teils (2) seitliche Ränder (9) aufweist, die auf ihrer Rückseite (11) durch seitliche Rückführungen (7) aus Strahlenschutzmaterial verlängert sind, wobei die seitlichen Rückführungen (7) jeweils eine obere Klappe (7a) aufweisen, die um ein senkrechtes Gelenk (7b) beweglich ist, das entlang des seitlichen Rands (9) gegenüber der Frontwandung (6) des unteren Teils (2) eingerichtet ist, wobei die oberen Klappen (7a) zwischen einer geschlossenen Stellung, in der sie sich in der Ebene oder im Wesentlichen in der Ebene der seitlichen Rückführungen (7) befinden, und einer offenen Stellung, in der sie dazu ausgelegt sind, sich in der Ebene oder im Wesentlichen in der Ebene der Frontwandung (6) des unteren Teils (2) zu befinden, bewegbar sind.

6. Schirm (1) gemäß den miteinander kombinierten Ansprüchen 4 und 5, **dadurch gekennzeichnet, daß** die seitlichen Stützen (17) entlang des hinteren Rands (14) der seitlichen Rückführungen (7) des unteren Teils (2) eingerichtet sind.

7. Schirm (1) gemäß einem der Ansprüche 1 bis 6, **dadurch gekennzeichnet, daß** die Frontwandung (20) des oberen Teils (3) eine aus durchsichtigem Strahlenschutzmaterial gefertigte obere Platte (26) aufweist, die, wenn sie in der aktiven Stellung ist, gegenüber der Frontwandung (6) des unteren Teils (2) nach vorne geneigt ist, also einen Überhang bildet, um dem Bediener zu ermöglichen, sich dem Einsatzgebiet zu nähern.

8. Schirm (1) gemäß Anspruch 7, **dadurch gekennzeichnet, daß** die obere Platte (26) des oberen Teils (3) nach hinten durch eine aus Strahlenschutzmaterial gefertigte Deckplatte (28) verlängert ist, wobei die seitlichen Ränder (23, 30) der oberen Platte (26) und der Deckplatte (28) nach hinten durch seitliche Rückführungen (31) aus Strahlenschutzmaterial verlängert sind.

9. Schirm (1) gemäß den miteinander kombinierten Ansprüchen 3 und 8, **dadurch gekennzeichnet, daß** das untere Ende der seitlichen Rückführungen (31) des oberen Teils (3) einen flexiblen Vorhang (36) aus Strahlenschutzmaterial aufweist, um in Verlängerung des zwischen den Frontwandungen (6 und 20) des unteren (2) beziehungsweise des oberen (3) Teils eingerichtete seitliche Durchgänge für die Arme des Bedieners zu bilden.

10. Schirm (1) gemäß einem der Ansprüche 8 oder 9, **dadurch gekennzeichnet, daß** die seitlichen Rückführungen (31) des oberen Teils (3) jeweils eine um ein Gelenk (32b) bewegbare untere Klappe (33) aufweisen, wobei die Klappen (33) die flexiblen Vorhänge (36) aufweisen und zwischen einer geschlossenen Stellung, in der sie sich in der Ebene oder im Wesentlichen in der Ebene der seitlichen Rückführungen (31) befinden, und einer offenen Stellung, in der sie nach außen geklappt sind, bewegbar sind.

11. Schirm (1) gemäß den miteinander kombinierten Ansprüchen 1, 4 und 8, **dadurch gekennzeichnet, daß** der obere Teil (3) mit dem unteren Teil (2) durch um eine ein Drehgelenk bildende, im Bereich des oberen Endes (19) der seitlichen Stützen (17) angeordnete horizontale erste Gelenkachse (38) gelenkige seitliche Arme (37b) fest verbunden ist, wobei die seitlichen Arme (37b) mit den seitlichen Rückführungen (31) des oberen Teils (3) durch eine ein Drehgelenk bildende, oberhalb der ersten Gelenkachse (38) angeordnete horizontale zweite Gelenkachse (42) fest verbunden sind, wobei die Gelenke (38, 42) Mitteln (39, 43) zum Steuern der Schwenkbewegungen, zum Beispiel vom Typ eines Kolbens, zugeordnet sind.

12. Schirm (1) gemäß einem der Ansprüche 1 bis 11, **dadurch gekennzeichnet, daß** die Frontwandung (6) des unteren Teils (2) aus einer starren, aus einem Strahlenschutzmaterial gefertigten Platte (2a) besteht, in der eine Öffnung (54) eingerichtet ist, die mit einem flexiblen Vorhang (55) in Form einer Anzahl senkrechter, nebeneinander angeordneter, aus einem Strahlenschutzmaterial gefertigter Bänder oder Streifen verschlossen ist, die das Hindurchreichen von Ausrüstungen oder Teilen von Ausrüstungen durch die Öffnung (54) ermöglicht.

13. Schirm (1) gemäß einem der Ansprüche 1 bis 12, **dadurch gekennzeichnet, daß** der untere Teil aus zwei komplementären, senkrecht gegeneinander verschiebbaren Teilen (2a, 2b) besteht, die sich teilweise überdecken, um eine Veränderung ihrer Höhe zu ermöglichen, wobei die beiden Teile (2a, 2b) durch Mittel in Form von Führungen oder Gleitschienen miteinander verbunden sind, die einem oder mehreren Betätigungselementen (52) zugeordnet sind, die ermöglichen, die jeweilige Stellung der beiden den Teil bildenden Teile anzupassen.

14. Ausstattung in Form einer sterilen Hülle, die dazu bestimmt ist, wenigstens einen Teil der Höhe eines Schirms (1) gemäß den miteinander kombinierten Ansprüchen 5 und 10 zu bedecken, wobei die Ausstattung
- eine flexible Tasche (48), die dazu ausgelegt ist, das untere Ende der Frontwandung (20) des oberen Teils (3) zu bedecken, und die mit Mitteln für deren Befestigung an der Frontwandung (20) versehen ist,
- zwei flexible Taschen (49), die dazu ausgelegt sind, die beweglichen unteren Klappen (33) der seitlichen Rückführungen (31) des oberen Teils (3) zu bedecken, und die mit Mitteln für deren Befestigung an der zugehörigen beweglichen Klappe (33) versehen sind,
- eine flexible Tasche (50), die dazu ausgelegt ist, das obere Ende der Frontwandung (6) des unteren Teils (2) und die seitlichen Klappen (7a) der seitlichen Rückführungen (7) zu bedecken, und die mit Mitteln für deren Befestigung an der Frontwandung (6) und den Klappen (7a) versehen ist, und
- zwei Strukturen (51) zum Verkleiden der seitlichen Stützen (17) und eventuell eines Teils der Rückführungen (7), die mit Mitteln für deren Befestigung an den seitlichen Stützen (7) und eventuell den Rückführungen (7) versehen sind, aufweist.

## Claims

1. Screen (1) made of a radioprotective material for protecting an operator from the ionising radiations, which screen (1) comprises:
(i) a lower part (2) that has a base (4), and at least one front wall (6) made of a radioprotective material, which front wall (6) extends in a vertical or substantially vertical plane and has an upper edge (8), a front face (10) and a rear face (11),
(ii) an upper part (3) having at least one front part (20) made of a radioprotective material, at least one part (26) of which is transparent to allow a visual access through it, which front wall (20) has a front face (24), a rear face (25) and a lower edge (22), and
(iii) at least one passage (27) for the operator's arms,
said front wall (6) of the lower part (2) and said front wall (20) of the upper part (3) being arranged in the continuation of each other, by juxtaposition of their respective upper (8) and lower (22) edges, then occupying an active position ensuring the desired protection, said front walls (6, 20) of the upper part (3) and of the lower part (2) being separable from each other,
**characterised in that** said upper part (3) is carried by said lower part (2), through linking means (17, 38, 37, 42) allowing the reversible operations thereof between said active position and a retracted position in which the lower edge (22) of said front wall (20) of said upper part (3) is spaced apart from the upper edge (8) of said front wall (6) of said lower part (2), to clear a space above said lower part (2),
said linking means (17, 38, 37, 42) including means for the pivoting operation (38, 42) of said upper part (3) with respect to said lower part (2), about at least one horizontal axis (38, 42).

2. Screen (1) according to claim 1, **characterized in that** said passage (27) for the operator's arms is arranged at the level of the zone of juxtaposition between said lower (2) and upper (3) parts, when these latter are in an active juxtaposed position.

3. Screen (1) according to claim 2, **characterized in that** said passage for the operator's arms is arranged at the level of the lower edge of the front wall (20) of the upper part (3) and consists in a flexible curtain (27) in the form of a plurality of vertical strips or bands juxtaposed to each other, made of a radioprotective material, and the upper end of which is fixed on an upper panel (26) made of a radioprotective material.

4. Screen (1) according to any one of claims 1 to 3, **characterized in that** the upper part (3) is mounted at the end of two lateral uprights (17) fastened to the lower part (2), which uprights (17) are offset rearwardly with respect to the plane of the front wall (6) of said lower part (2).

5. Screen (1) according to any one of claims 1 to 4, **characterized in that** said front wall (6) of the lower part (2) has lateral edges (9) that are continued, on the side of its rear face (11), by lateral returns (7) made of a radioprotective material,
said lateral returns (7) each having an upper flap (7a) mobile about a vertical articulation (7b) arranged along the lateral edge (9) opposite of the front wall (6) of the lower part (2), which upper flaps (7a) are mobile between a closed position in which they are located in or substantially in the plane of said lateral returns (7), and an open position in which they are adapted to be located in or substantially in the plane of said front wall (6) of the lower part (2).

6. Screen (1) according to claims 4 and 5, taken in combination, **characterized in that** said lateral uprights (17) are arranged along the rear edge (14) of the lateral returns (7) of the lower part (2).

7. Screen (1) according to any one of claims 1 to 6, **characterized in that** the front wall (20) of the upper part (3) includes an upper panel (26) made of a transparent radioprotective material that, once in active position, is inclined forwardly with respect to the front wall (6) of the lower part (2), forming an overhanging, to allow the operator to move closer to the zone of intervention.

8. Screen (1) according to claim 7, **characterized in that** said upper panel (26) of the upper part (3) is continued rearwardly by a covering panel (28) made of a radioprotective material, the lateral edges (23, 30) of said upper panel (26) and of said covering panel (28), being continued, rearwardly, by lateral returns (31) made of a radioprotective material.

9. Screen (1) according to claims 3 and 8, taken in combination, **characterized in that** the lower end of the lateral returns (31) of said upper part (3) comprise a flexible curtain (36) made of a radioprotective material, so as to create lateral passages for the operator's arms, in the continuation of said passage (27) arranged between said front walls (6 and 20) of the lower (2) and upper (3) parts.

10. Screen (1) according to any one of claims 8 or 9, **characterized in that** said lateral returns (31) of said upper part (3) each include a lower flap (33) mobile about an articulation (32b), which flaps (33) include said flexible curtains (36) and are mobile between a closed position in which they are located in or substantially in the plane of said lateral returns (31), and an open position in which they are extended outwardly.

11. Screen (1) according to claims 1, 4 and 8, taken in combination, **characterized in that** the upper part (3) is fastened to the lower part (2) through lateral arms (37b) articulated about a first horizontal axis of articulation (38), forming a pivot, located at the upper end (19) of the lateral uprights (17), which lateral arms (37b) are fastened to the lateral returns (31) of said upper part (3), through a second horizontal axis of articulation (42), forming a pivot, located above said first axis of articulation (38), which articulations (38, 42) are associated with means for controlling the pivoting movements (39, 43), of the cylinder type for example.

12. Screen (1) according to any one of claims 1 to 11, **characterized in that** the front wall (6) of the lower part (2) consists in a rigid panel made of a radioprotective material (2a), in which is formed an opening (54) closed by a flexible curtain (55) in the form of a plurality of vertical strips of bands juxtaposed to each other, made of a radioprotective material, allowing the passage of devices or parts of devices though said opening (54).

13. Screen (1) according to any one of claims 1 to 12, **characterized in that** the lower part is consisted of two complementary parts (2a, 2b) sliding vertically relative to each other, and which partially overlap each other to allow a variation of its height, which two parts (2a, 2b) are assembled to each other by means in the form of guides or slides associated with one or several actuator(s) (52) allowing to adapt the respective position of said two constitutive parts.

14. Equipment in the form of a sterile cover intended to cover at least one part of the height of a screen (1) according to claims 5 and 10, taken in combination, the equipment comprising:
- a flexible envelope (48) adapted to come and cover the lower end of the front wall (20) of the upper part (3) and provided with means for the fixation thereof to said front wall (20),
- two flexible envelopes (49) adapted to come and cover the mobile lower flaps (33) of the lateral returns (31) of the upper part (3), provided with means for the fixation thereof to said associated mobile flap (33),
- a flexible envelope (50) adapted to come and cover the upper end of the front wall (6) of the lower part (2) and the upper flaps (7a) of the associated lateral returns (7), provided with means for the fixation thereof to said front wall (6) and said flaps (7a), and
- two structures (51) for covering the lateral uprights (17), with possibly a part of the returns (7), provided with means for the fixation thereof to said lateral uprights (17) and possibly the returns (7).
